(19) Europäisches Patentamt
European Patent Office
Office européen des brevets

(11) **EP 3 531 426 A1**

(12) **EUROPEAN PATENT APPLICATION**

(43) Date of publication:
**28.08.2019 Bulletin 2019/35**

(51) Int Cl.:
***G16H 30/40*** *(2018.01)*    ***G16H 50/50*** *(2018.01)*

(21) Application number: **18158410.3**

(22) Date of filing: **23.02.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Charité - Universitätsmedizin Berlin
10117 Berlin (DE)**

(72) Inventors:
• **Kelm, Marcus
13127 Berlin (DE)**

• **Goubergrits, Leonid
12357 Berlin (DE)**
• **Fernandes, Joao Filipe
1500-140 Lisboa (PT)**
• **Kühne, Titus
14129 Berlin (DE)**

(74) Representative: **Hofmann, Matthias
Boehmert & Boehmert
Anwaltspartnerschaft mbB
Pettenkoferstraße 22
80336 München (DE)**

(54) **NON-INVASIVE MEASUREMENT OF MYOCARDIAL POWER**

(57) A method for determining an internal myocardial power of a heart, the method comprising non-invasively obtaining one or more first images that are indicative of a structure of the heart, non-invasively obtaining one or more second images that are indicative of a flow property of the heart, segmenting, based on the one or more first images, the blood pool and the myocardium of the heart, determining, based on the segmented blood pool, the myocardium and the one or more second images, one or more mechanical parameters of the heart, and determining the internal myocardial power based on the one or more mechanical parameters.

non-invasively obtaining a first plurality of images that are indicative of a structure of the heart and non-invasively obtaining a second plurality of images that are indicative of a flow property of the heart — 110

segmenting, based on the first plurality of images, the blood pool and the myocardium of the heart — 120

determining, based on the segmented blood pool, the myocardium and the second plurality of images, one or more mechanical parameters of the heart — 130

determining the internal myocardial power based on the mechanical parameters — 140

100

**FIG. 1**

EP 3 531 426 A1

**Description**

**TECHNICAL FIELD**

The present invention relates to methods and devices for measuring myocardial power. The present invention also relates to a computer-readable storage medium storing program code, the program code comprising instructions for carrying out such a method.

**BACKGROUND**

In heart disease pressure-volume overload conditions trigger cardiac remodeling which causes concentric or eccentric hypertrophy. At longer-term, there can be a transition from adaptive hypertrophy to heart failure. In addition, arrhythmias with risk for sudden cardiac death can evolve. However, there is large variability how patients deal with overload. Furthermore, neither the onset of clinical symptoms nor the degree of hypertrophy (chamber size, wall thickness) is directly related to the degree of external load on the heart.

Therefore, energetic approaches for evaluating the myocardial performance are becoming increasingly of interest. There is growing evidence that in disease the myocardial efficiency is reduced this means that the myocardium will need more energy to pump a given amount of blood against a given resistance. Accordingly, new pharmacological or interventional therapeutic concepts aiming to enhance the myocardial efficiency and/ or reduce the energy demand could be proposed.

Myocardial energy demand and efficiency are affected, amongst others, by adaptive changes in myocardial mass, LV chamber size and interstitial fibrosis in order to maintain myocardial wall stress according the Law of Laplace.

Methods for measuring myocardial efficiency did not become part of clinical routine yet due to their invasiveness. Current clinical routine is focusing on the assessment of the external myocardial power without an assessment of the internal myocardial power and respectively myocardial efficiency. More recently, combined Magnetic Resonance Imaging (MRI) and Positron Emission Tomography (PET) have been used to describe the ratio of the myocardial external power and internal energy consumption. While PET itself is non-invasive, it can only be applied for selected clinical indications and involves exposure to ionizing radiation. Thus the method is not available for the clinical routine.

**SUMMARY OF THE INVENTION**

The objective of the present invention is to provide a method and a device for measuring myocardial power, wherein the method and the device overcome one or more of the above-mentioned problems of the prior art.

A first aspect of the present invention provides method for determining an internal myocardial power of a heart, the method comprising:

- non-invasively obtaining one or more first images that are indicative of a structure of the heart,
- non-invasively obtaining one or more second images that are indicative of a flow property of the heart,
- segmenting, based on the first images, the blood pool and the myocardium of the heart,
- determining, based on the segmented blood pool, the myocardium and the second images, one or more mechanical parameters of the heart, and
- determining the internal myocardial power based on the one or more mechanical parameters.

The method of the first aspect allows determining an internal myocardial power of the heart without exposing the patient to radiation and without requiring invasive procedures such as placing a catheter. Experiments have shown that the method of the first aspect nonetheless provides clinically reliable accuracy.

Thus, diagnostic measures of myocardial function based on non-invasive imaging data, which could be acquired in clinical routine.

Non-invasively obtaining images may include obtaining images over a network that have previously been acquired using a non-invasive imaging method (e.g. MRI or CT). Due to the required injection of a tracer, PET shall herein be considered as an invasive imaging method.

The one or more mechanical parameters of the heart may be any kind of parameters of the heart or a surrounding of the heart that relate to any kind of mechanical properties, e.g. a size, a volume, a duration, a duration of a periodic movement, a contraction time of a vehicle, an inflow closing time, or any other parameters relating to measured times or velocities of elements of the heart.

The first images may comprise one or more images of the heart at a first point in time and one or more images of the heart at a second point in time. Segmenting the first images may comprise performing a first segmentation of the images of the first point in time and performing a second segmentation of the images of the second point in time. These first, second (and potentially many further segmentations) may in the following just be referred to as "the segmentation".

In embodiments, the internal myocardial power may also refer to a circulatory power of the heart.

In a first implementation of the method of the first aspect, the method further comprises during the non-invasive acquisition of the first and second images, acquiring a heart rate and/or a systemic blood pressure. This has the advantage that the myocardial power can be determined with higher accuracy.

In a further implementation,

- the first images are acquired using MRI, CT and/or echocardiography, and/or
- the second images are acquired using 4D flow MRI, 2D flow MRI and/or Doppler echocardiography.

These imaging modalities have proven particularly useful for implementing the method of the first aspect.

In a further implementation, the one or more mechanical parameters include a contraction time of the heart, wherein the contraction time is determined based on a sum of an inflow valve closing time, an ejection time, and an isovolumetric contraction time.

Preferably, the inflow valve closing time is determined as a time when a first and a second pressure curve intersect, wherein preferably the first pressure curve is modeled as a hyperbola and the second curve is modeled as a parabola, the ejection time is determined based on an outflow curve that is determined based on the second images, and/or the isovolumetric contraction time is determined based on a combined inflow and outflow curve.

In a further implementation, the one or more mechanical parameters include a ventricular radius, a length, a volume, a myocardial volume, and/or a myocardial wall thickness of the heart.

In a further implementation, the internal myocardial power that is determined based on a volume of the myocardial wall volume $V_{wall}$, a wall stress $\sigma_{wall}$, and a contraction time of the ventricle $t_{cs}$, preferably based on the equation

$$IMP = \frac{V_{wall} * \sigma_{wall}}{t_{CS}}.$$

This equation is easy to calculate, yet provides an accurate estimate of the internal myocardial power.

In a further implementation, the method further comprises determining a circulatory power based on a mean arterial pressure and an effective cardiac output, preferably based on a product of mean arterial pressure and effective cardiac output.

In a further implementation, the one or more mechanical parameters include a myocardial volume, a ventricular wall stress, a peak systolic pressure, a forward flow value and/or a backward flow volume.

A second aspect of the present invention provides device for determining an internal myocardial power of a heart based on non-invasive imaging, the device comprising:

- an image obtaining unit configured to obtain one or more first images of the heart that are indicative of a structure of the heart and one or more second images that are indicative of a flow property of the heart,
- a segmentation unit configured to segment, based on the one or more first images, the blood pool and the myocardium of the heart,
- a parameter determining unit configured to determine, based on the segmented blood pool and the myocardium and the one or more second images, one or more mechanical parameters, and
- a calculation unit configured to determine the internal myocardial power based on the mechanical parameters.

In a first implementation of the second aspect, the image obtaining unit of the device comprises a network interface configured to obtain the first images and/or the second images from a remote device in the network, in particular from an MRI, CT or echocardiography machine.

In a second implementation of the device of the second aspect, the device further comprises a user interface for a visual verification of the segmentation by a physician. This has the advantage that potentially

A third aspect of the present invention provides computer-readable storage medium storing program code, the program code comprising instructions that when executed by a processor carry out the method of the first aspect or one of its implementations.

Embodiments of the invention relate to the non-invasive diagnostic procedure characterizing individually myocardial internal and external power as well as respective myocardial power efficiency. In particular, embodiments relate to assessing the reduced myocardial efficiency due to congenital or acquired heart diseases such as coarctation of the aorta, bicuspid aortic valves, aortic or mitral valve stenosis and aortic or mitral valve regurgitation. These diseases cause additional load for the heart that could be initially compensated by the heart. With a disease development, heart remodeling is initiated and the myocardial efficiency is decreasing thus reducing the ability of the heart to provide additional power due to additional load caused by diseases as well as additional power required during stress conditions. An assessment of the myocardial efficiency, which requires an assessment of both internal and external myocardial power, allows assessing the individual response of the heart to the disease and thus could support time and type (interventional and/or pharmacological) of a treatment decision of a physician.

## BRIEF DESCRIPTION OF THE DRAWINGS

To illustrate the technical features of embodiments of the present invention more clearly, the accompanying drawings provided for describing the embodiments are introduced briefly in the following. The accompanying drawings in the following description are merely some embodiments of the present invention, modifications on these embodiments are possible without departing from the scope of the present invention as defined in the claims.

FIG. 1      is a block diagram illustrating a method in accordance with an embodiment of the present invention,

FIG. 2      is a block chart illustrating a number of optional features of embodiments of the present invention,

FIG. 3      is a schematic illustration of parameters used to estimate contraction time in accordance with the present invention,

FIG. 4      is a schematic illustration of a method to estimate inflow valve closing time in accordance with the present invention,

FIG. 5      is a diagram illustrating internal myocardial power values that have been determined with a method according to the present invention, and

FIG. 6      is a diagram illustrating external myocardial efficiency values and circulatory efficiency values that have been determined with a method according to the present invention.

## DETAILED DESCRIPTION OF EMBODIMENTS

The following description illustrates preferred embodiments of the present invention, the scope of the present invention is not limited to this. Any variations or replacements can be easily made through person skilled in the art. Therefore, the protection scope of the present invention should be subject to the protection scope of the attached claims.

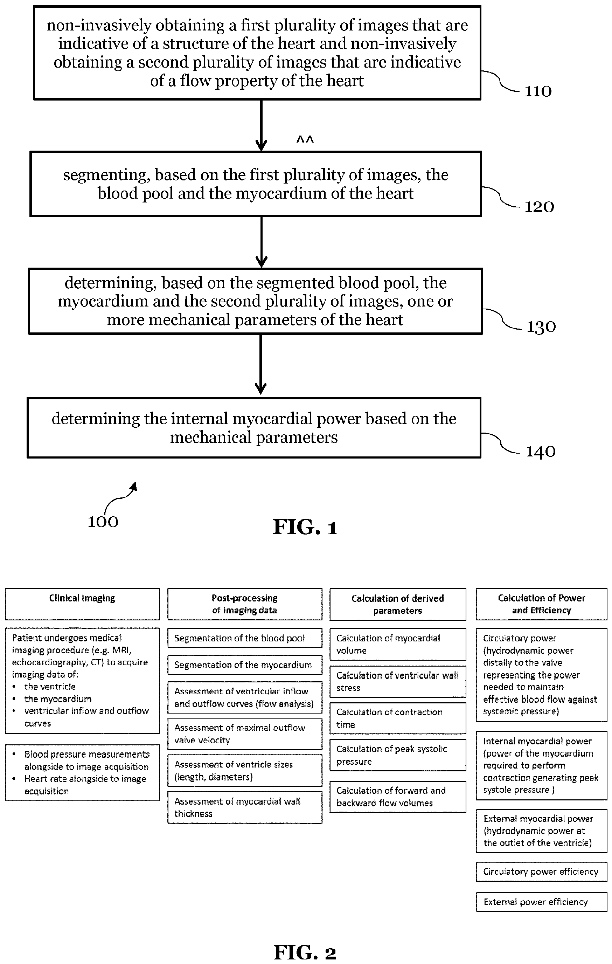

FIG. 1 is a block diagram illustrating a method in accordance with an embodiment of the present invention.

The method comprises a first step of non-invasively obtaining one or more first images that are indicative of a structure of the heart and non-invasively obtaining one or more second images that are indicative of a flow property of the heart. For example, the obtained images may be images that have been previously acquired using non-invasive images and stored on a workstation.

In a second step of the method, based on the one or more first images, the blood pool and the myocardium of the heart are segmented. It is understood that the segmentation of the blood pool and the myocardium may be also be based on further information, e.g. from the one or more second images.

In a third step, based on the segmented blood pool, the myocardium and the second images, one or more mechanical parameters of the heart are determined.

In a fourth step, the internal myocardial power is determined based on the one or more mechanical parameters.

FIG. 2 shows a block chart for diagnostic measures including four steps: clinical imaging accompanied with an assessment of heart rate and systemic blood pressures, post-processing of imaging data for anatomy and flow, calculation of derived parameters based on post-processing and finally calculation of the myocardial power and efficiency for clinical measures. The steps shall be explained in more detail in the following.

### Clinical imaging

Patients undergo imaging procedure allowing to acquire imaging data of the ventricle blood pool, myocardium as well as ventricular inflow and outflow conditions. MRI, CT and echocardiography or their combination are, among others, possible imaging modalities. 3D data are preferable. However, analysis could also be done based on 2D imaging data. Flow conditions can be assessed directly by methods like 4D MRI or Doppler echocardiography. Alternatively, they could be assessed indirectly by volumetric measurements of the ventricle (e.g. from 3D echocardiography or CINE MRT). Imaging acquisition is preferably accompanied by an assessment of the heart rate (HR) as well as the measurement of systemic blood pressure: 1) preferably using non-invasive methods (e.g. cuff-based measurements) or 2) can be performed based on invasive catheter-based measurements.

**Post-processing of imaging data**

Post-processing can be performed by various interchangeable software tools. Several products are regularly available for routinely-used imaging devices or independent solutions can be used. The segmentation of the blood pool and the myocardium needs to be performed. Based on segmentations geometrical parameters of the ventricle and myocardium necessary to calculate the ventricular wall stress are assessed: ventricular radii, lengths and volumes, myocardial volume, myocardial wall thickness. Flow analysis needs to be performed: If no direct ventricular pressure measurement is available, inflow and outflow curves should be generated in order to measure contraction time. Flow curves could be measured directly based on flow imaging or indirectly based on dynamic assessment of geometries. If no direct ventricular pressure measurement is available, maximal outflow valve velocity should be measured to allow estimation of the valve pressure drop.

**Calculation of derived parameters**

Derived parameters based on post-processed data are calculated: Myocardial volume is derived from myocardial wall segmentation or based on ventricular length together with myocardial wall thickness. If peak systolic ventricular pressure $P_{sys}$ is not measured directly, $P_{sys}$ should be derived indirectly. $P_{sys}$ is the calculated sum of the systolic pressure in the aorta and the pressure gradient across the outflow valve, whereas the systolic pressure in the aorta is derived from the peripherally (e.g. arm) measured systolic pressure by taking into account the increasing from the heart pulse pressure. The pulse pressure increase could be calculated using in the literature published equations. The valve pressure drop is calculated by using the Bernoulli's equation. Ventricular wall stress $\sigma_{wall}$ is calculated based on measured ventricular peak systolic pressure and geometric parameters of the myocardial wall. The simplest way is the calculation according to the Laplace law:

$$\sigma_{wall} = P_{sys} * \frac{R_{BP}}{2 * S_{wall}}$$

where $S_{wall}$ is a mean myocardial wall thickness, $P_{sys}$ is the peak systolic pressure in the ventricle and $R_{BP}$ is the mean radius of the ventricular blood pool. The wall stress could also be calculated using other possible equations (e.g. thick wall Laplace law). These other equations may be based on the same kind of parameters, including $S_{wall}$, $P_{sys}$, and $R_{BP}$. Contraction time ($t_{cs}$) is the period between the start of ventricular contraction and peak systolic pressure and/or flow. If contraction time is not measured directly from ventricular pressure curves, tcs should be derived indirectly: The sum of inflow valve closing time $t_{CT}$, isovolumetric contraction time $t_{IVC}$ and ejection time $t_{ET}$ define $t_{cs}$. The ejection time $t_{ET}$ is derived from outflow curve. The isovolumetric contraction time $t_{IVC}$ is derived from combined inflow and outflow curves. The sum of the inflow valve closing time $t_{CT}$ and isovolumetric contraction time $t_{IVC}$ is derived from ejection time $t_{ET}$ together with systemic pressure values assuming ventricular pressure increase according to parabolic equation. In order to calculate internal myocardial power, the partial use of individual components ($t_{cs}$, $t_{IVC}$, $t_{ET}$) may provide further simplification with adequate diagnostic accuracy. Forward and backward flow volumes are derived from outflow curves or based on dynamic assessment of ventricular geometry.

**Non-invasive assessment of contraction time**

In the following, a preferred way of determining the contraction time $t_{cs}$ shall be explained in detail. Therein, the contraction time $t_{cs}$ is defined as the period between the start of ventricular (myocardial) contraction at the end-diastolic pressure and a peak systolic pressure which is correlating with a peak aortic flow.

A known direct way of measuring the contraction time is based on catheter measured pressure curves that represent an invasive measurement associated with X-Ray radiation as well as a use of contrast agent. However, these are both associated with a risk for patients.

Instead, an indirect non-invasive approach allows determining the contraction time $t_{cs}$ as the sum of three sequent periods: inflow valve closing time $t_{CT}$, isovolumetric contraction time $t_{IVC}$ and ejection time $t_{ET}$:

$$(1) \qquad t_{CS} = t_{CT} + t_{IVC} + t_{ET}$$

The inflow valve closing time $t_{CT}$ is a period of the increasing ventricle pressure necessary to stop the blood inflowing from the atrium into the ventricle, which is owning a momentum. The inflow valve closing time $t_{CT}$ can be determined as a time at which two curves, a hyperbola and a parabola, are crossing. Therein, a hyperbola is a pressure curve $p_H$ as a function of time t, [ms], e.g. as described by:

$$(2) \qquad p_H = (25^*1.05^*(SV)^{2/3}*10^5) / (133^*T_{dia}^*t), [mmHg]$$

whereas SV is a stroke volume [ml] and $T_{dia}$ is a diastole period [ms] calculated from heart rate HR [bpm] using e.g. the following equation:

$$(3) \qquad T_{dia} = (60^*10^3/HR) - 805^*(0.18365317 + 0.54677315^*e^{(-0.015150054^*HR)})$$

The used parabola curve can be a pressure curve $p_p$ as a function of time t, [ms] as described by:

$$(4) \qquad p_p = P_{SYS} - C^*(t - T_1)^2, [mmHg]$$

whereas time $T_1$, [ms] is calculated e.g. according to following equation (5) and a parabola constant C e.g. according to following equation (6):

$$(5) \qquad T_1 = t_{ET} + t_{ET}^*(P_{SYS}/(P_{SYS}-P_{DIA}))^{1/2}$$

$$(6) \qquad C = (P_{SYS}-P_{DIA})/ (t_{ET})^2$$

$P_{SYS}$ and $P_{DIA}$, [mmHg] are systolic and diastolic pressures. The ejection time $t_{ET}$ is directly measured from outflow curve. The parabola equation is fully defined by three parameters: two pressure values $P_{SYS}$ and $P_{DIA}$ of the parabola based on cuff based measurements and a period $t_{ET}$ between these two parabola points as measured from flow curve as well as an assigning of the point $P_{SYS}$ to the parabola vertex.

The isovolumetric contraction time $t_{IVC}$ can be directly measured from combined inflow and outflow curves.

Alternatively, the sum of the inflow valve closing time $t_{CT}$ and isovolumetric contraction time $t_{IVC}$ could be estimated from ejection time $t_{ET}$ together with systemic pressure values $P_{SYS}$ and $P_{DIA}$, assuming ventricular pressure increase according to parabolic equation (4).

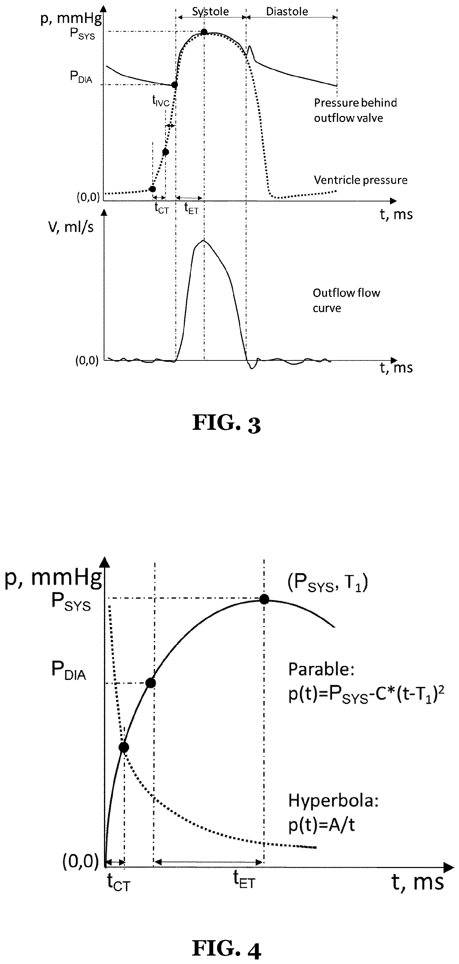

FIG. 3 provides a further illustration of parameters used to estimate the contraction time and FIG. 4 illustrates a method for estimating the inflow valve closing time. As illustrated in FIG. 4, the inflow valve closing time may be defined as a time when a first and a second pressure curve intersect, wherein the first pressure curve is modeled as a hyperbola $p(t) = A/t$, wherein $A$ is a predetermined value, e.g. determined based on $(SV)^{2/3}$, and the second curve is modeled as a parabola $p(t) = P_{SYS} - C^* (t - T_1)^2$, wherein $P_{SYS}$ is the peak systolic pressure and $C$ and $T_1$ are constant values, e.g. determined according to equations (5) and (6).

**Calculation of Power and Efficiency**

Internal myocardial power is defined as the power of the heart to perform myocardial contraction. Internal myocardial power IMP is preferably calculated using the following equation:

$$IMP = \frac{V_{wall} * \sigma_{wall}}{t_{CS}}$$

wherein $V_{wall}$ is the myocardial wall volume, $\sigma_{wall}$ the wall stress, and $t_{cs}$ the contraction time of the ventricle. Internal myocardial power is indexed to BSA allowing inter-individual comparison.

Circulatory Power (CP) is defined as the hydrodynamic power after the ventricle outflow valve. The power needed to pump a given amount of blood against a given resistance characterized by systemic pressure. CP is calculated with the following equation:

$$CP = MAP * Q[W]$$

Therein, the mean arterial pressure (MAP) is assessed from measured peripheral systolic and diastolic pressure. Q is the effective Cardiac Output ($CO_{eff}$) and can be assessed from heart volumetry: $CO_{eff}$ = (forward flow volume - backward flow volume) * HR (heart rate) or from outflow curves, depending on the imaging modalities used. The ratio between CP and IMP

$$\text{Eff}_{CP} = \frac{CP}{IMP} * 100[\%]$$

is defined as the Circulatory Efficiency ($Eff_{CP}$) of the heart. $Eff_{CP}$ is the level of efficiency of the myocardium to provide effective cardiac output in the arterial system taking into account power loss in the myocardium and due to diseased heart valves.

External Myocardial Power is defined as hydrodynamic power at the outlet of the ventricle. It is the power needed to pump a given amount of blood against a given resistance and across a given gradient by a stenotic valve. EMP is computed by the following equation: EMP = (MAP + AoG) * $CO_{total}$. AoG is the aortic pressure gradient and $CO_{total}$ (forward flow volume) multiplied by HR. The external myocardial efficiency is defined as ratio between EMP and IMP: is defined as the External Myocardial efficiency ($Eff_{EMP}$) of the heart:

$$\text{Eff}_{EMP} = \frac{EMP}{IMP} * 100[\%]$$

It is the level of efficiency of the myocardium to provide effective cardiac output to arterial system and, additionally, to overcome all resistances "such as the pressure gradients across stenotic valve.

**Exemplarily use of the heart power and efficiency analysis**

The disclosed method can be applied in patients with different heart disease. Myocardial power was successfully shown to be able to quantify cardiac remodeling processes. Circulatory efficiency and internal myocardial power measurements gradually reflect disease response in patients with aortic valve stenosis (AS) and combine aortic valve stenosis and insufficiency (AS/AI), even when external myocardial efficiency and EF are still compensated.

In patients with AS (n=59), AS/AR (n=21) and controls (n=14) internal myocardial power, external myocardial efficiency and circulatory efficiency were computed from MRI volumetric as well as blood flow measurements and blood pressures. Data were compared between groups; sub-analysis was performed for patients with normal and reduced ejection fraction (EF). Median internal myocardial power was increased in AS, 7.7W/m2 (interquartile range [IQR] 6.0-10.2; p=0.002) and AS/AR, 10.8W/m2 (8.9-13.4; p<0.001) when compared to controls, 5.4W/m2 (4.2-6.5), and was lower in AS than AS/AR (p<0.001). Circulatory efficiency was decreased in AS, 8.6% (6.8-11.1; p=0.008) and AS/AR, 5.4% (4.1-6.2; p<0.001) when compared to controls, 11.8% (9.8-16.9), and was higher in AS than AS/AR (p<o.ooi).

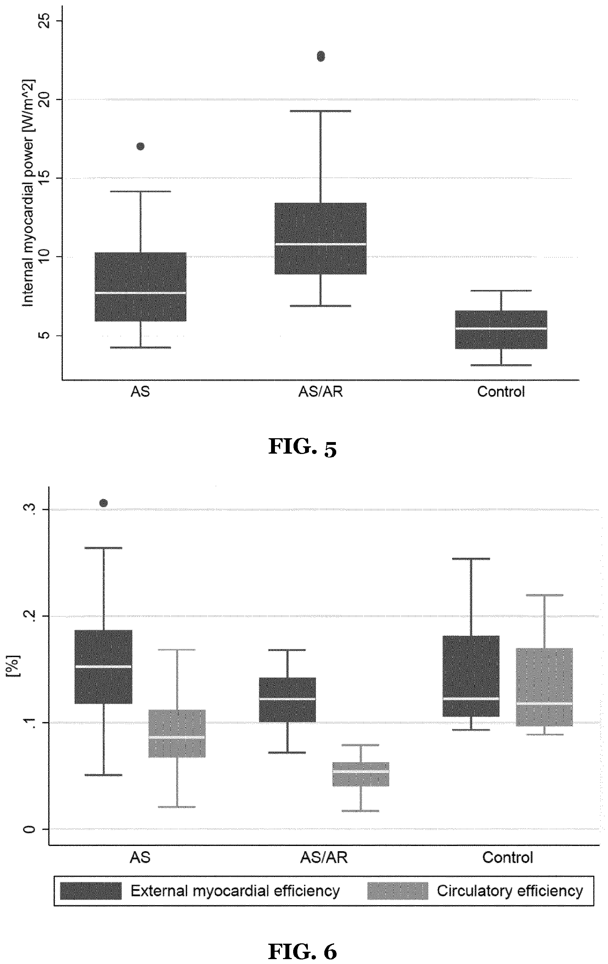

External myocardial efficiency was significantly higher in AS, 15.2% (11.9-18.6) than in AS/AR, 12.2% (10.1-14.2; p=0.011). External myocardial efficiency did not differ in patients with normal EF, 14.9% (12.9-17.9) when compared to controls 12.2% (10.7-18.1; p=0.218) and was significantly lower in patients with reduced EF, 9.8% (8.1-11.7; p=0.025). The results are illustrated in FIG. 5 and FIG. 6.

**Claims**

1. A method (100) for determining an internal myocardial power of a heart, the method comprising:

   - non-invasively obtaining (110) one or more first images that are indicative of a structure of the heart,
   - non-invasively obtaining (110) one or more second images that are indicative of a flow property of the heart,
   - segmenting (120), based on the one or more first images, the blood pool and the myocardium of the heart,
   - determining (130), based on the segmented blood pool, the myocardium and the one or more second images, one or more mechanical parameters of the heart, and
   - determining (140) the internal myocardial power based on the one or more mechanical parameters.

2. The method of claim 1, further comprising, during a non-invasive acquisition of the first and second images, acquiring a heart rate and/or a systemic blood pressure.

3. The method of one of the previous claims, wherein

   - the one or more first images are acquired using MRI, CT and/or echocardiography, and/or
   - the one or more second images are acquired using 4D flow MRI, 2D flow MRI and/or Doppler echocardiography.

4. The method of one of the previous claims, wherein the one or more mechanical parameters include a contraction time of the heart, wherein the contraction time is determined based on a sum of an inflow valve closing time, an ejection time, and an isovolumetric contraction time.

5. The method of claim 4, wherein

   - the inflow valve closing time is determined as a time when a first and a second pressure curve intersect, wherein preferably the first pressure curve is modeled as a hyperbola and the second curve is modeled as a parabola,
   - the ejection time is determined based on an outflow curve that is determined based on the one or more second images, and/or
   - the isovolumetric contraction time is determined based on a combined inflow and outflow curve.

6. The method of one of the previous claims, wherein the one or more mechanical parameters include a ventricular radius, a length, a volume, a myocardial volume, and/or a myocardial wall thickness of the heart.

7. The method of one of the previous claims, wherein the internal myocardial power that is determined based on a volume of the myocardial wall volume $V_{wall}$, a wall stress $\sigma_{wall}$, and a contraction time of the ventricle $t_{cs}$, preferably based on the equation

$$IMP = \frac{V_{wall} * \sigma_{wall}}{t_{cs}}.$$

8. The method of one of the previous claims, further comprising determining a circulatory power based on a mean arterial pressure and an effective cardiac output, preferably based on a product of mean arterial pressure and effective cardiac output.

9. The method of one of the previous claims, wherein the one or more mechanical parameters include a myocardial volume, a ventricular wall stress, a peak systolic pressure, a forward flow value and/or a backward flow volume.

10. A device for determining an internal myocardial power of a heart based on non-invasive imaging, the device comprising:

    - an image obtaining unit configured to obtain one or more first images of the heart that are indicative of a structure of the heart and one or more second images that are indicative of a flow property of the heart,
    - a segmentation unit configured to segment, based on the one or more first images, the blood pool and the myocardium of the heart,
    - a parameter determining unit configured to determine, based on the segmented blood pool and the myocardium and the one or more second images, one or more mechanical parameters, and
    - a calculation unit configured to determine the internal myocardial power based on the mechanical parameters.

11. The device of claim 10, wherein the image obtaining unit comprises a network interface configured to obtain the first images and/or the second images from a remote device in the network, in particular from an MRI, CT or echocardiography machine.

12. The device of claim 10 or 11, further comprising a user interface for a visual verification of the segmentation by a physician.

13. A computer-readable storage medium storing program code, the program code comprising instructions that when executed by a processor carry out the method of one of claims 1 to 9.

non-invasively obtaining a first plurality of images that are indicative of a structure of the heart and non-invasively obtaining a second plurality of images that are indicative of a flow property of the heart

— 110

segmenting, based on the first plurality of images, the blood pool and the myocardium of the heart

— 120

determining, based on the segmented blood pool, the myocardium and the second plurality of images, one or more mechanical parameters of the heart

— 130

determining the internal myocardial power based on the mechanical parameters

— 140

100

## FIG. 1

| Clinical Imaging | Post-processing of imaging data | Calculation of derived parameters | Calculation of Power and Efficiency |
|---|---|---|---|
| Patient undergoes medical imaging procedure (e.g. MRI, echocardiography, CT) to acquire imaging data of:<br>• the ventricle<br>• the myocardium<br>• ventricular inflow and outflow curves | Segmentation of the blood pool | Calculation of myocardial volume | Circulatory power (hydrodynamic power distally to the valve representing the power needed to maintain effective blood flow against systemic pressure) |
| | Segmentation of the myocardium | Calculation of ventricular wall stress | |
| | Assessment of ventricular inflow and outflow curves (flow analysis) | | |
| | | Calculation of contraction time | |
| • Blood pressure measurements alongside to image acquisition<br>• Heart rate alongside to image acquisition | Assessment of maximal outflow valve velocity | Calculation of peak systolic pressure | Internal myocardial power (power of the myocardium required to perform contraction generating peak systole pressure ) |
| | Assessment of ventricle sizes (length, diameters) | | |
| | | Calculation of forward and backward flow volumes | |
| | Assessment of myocardial wall thickness | | External myocardial power (hydrodynamic power at the outlet of the ventricle) |
| | | | Circulatory power efficiency |
| | | | External power efficiency |

## FIG. 2

**FIG. 3**

**FIG. 4**

**FIG. 5**

**FIG. 6**

Europäisches Patentamt
European Patent Office
Office européen des brevets

# EUROPEAN SEARCH REPORT

Application Number

EP 18 15 8410

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/228190 A1 (GEORGESCU BOGDAN [US] ET AL) 11 August 2016 (2016-08-11)<br>* paragraph [0002] - paragraph [0005] *<br>* paragraph [0020] - paragraph [0052] *<br>----- | 1-13 | INV.<br>G16H30/40<br>G16H50/50 |
| X | US 2011/144967 A1 (ADIROVICH LEV [IL])<br>16 June 2011 (2011-06-16)<br>* paragraph [0009] - paragraph [0164] *<br>----- | 1-13 | |

TECHNICAL FIELDS
SEARCHED (IPC)

G16H

The present search report has been drawn up for all claims

| Place of search | Date of completion of the search | Examiner |
|---|---|---|
| Munich | 31 August 2018 | Menschner, Philipp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 531 426 A1**

## ANNEX TO THE EUROPEAN SEARCH REPORT
## ON EUROPEAN PATENT APPLICATION NO.

EP 18 15 8410

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2018

| Patent document cited in search report | | Publication date | Patent family member(s) | | Publication date |
|---|---|---|---|---|---|
| US 2016228190 | A1 | 11-08-2016 | NONE | | |
| US 2011144967 | A1 | 16-06-2011 | EP | 2329358 A2 | 08-06-2011 |
| | | | US | 2011144967 A1 | 16-06-2011 |
| | | | WO | 2010018542 A2 | 18-02-2010 |

EPO FORM P0459

For more details about this annex : see Official Journal of the European Patent Office, No. 12/82

14